# EUROPEAN PATENT SPECIFICATION

(11) **EP 4 376 714 B1**
(45) Date of publication and mention of the grant of the patent: **28.10.2026**
(21) Application number: 22754006.9
(22) Date of filing: 14.07.2022
(51) Int. Cl.: A61B 5/24, A61B 5/00, A61B 5/37

(54) **RETAINING DEVICE FOR RETAINING A WEARABLE MONITORING DEVICE**
HALTEVORRICHTUNG ZUM HALTEN EINER TRAGBAREN ÜBERWACHUNGSVORRICHTUNG
DISPOSITIF DE RETENUE POUR RETENIR UN DISPOSITIF DE SURVEILLANCE POUVANT ÊTRE PORTÉ

(30) Priority: 27.07.2021 US 202163226021 P
(43) Date of publication of application: 05.06.2024
(73) Proprietor: UNEEG Medical A/S, 3450 Alleroed (DK)
(72) Inventor: IPSEN, Klaus Sommer, 3540 Lynge (DK)
(74) Representative: Widex A/S
(86) International application number: PCT/EP2022/069708
(87) International publication number: WO 2023/006437

(56) References cited:
- WO-A1-2020/023681
- WO-A1-2021/154936
- US-A1- 2011 319 729
- US-A1- 2012 022 346
- US-A1- 2019 167 167
- US-A1- 2020 101 278

## Description

The present invention relates to a retaining device for retaining a wearable monitoring device, especially to a body region of a person. The monitoring device is preferably adapted for gathering a bio-electrical signal from that person. The invention relates also to a monitoring system.

Bio-electrical signals are here understood to be electrical potential differences across a tissue, organ or cell system. The best known examples for application or utilization of such signals are Electrocardiogram (ECG), Electroencephalogram (EEG) and Electromyogram (EMG). A (personal) wearable monitoring device denotes especially a device adapted for long term monitoring, e.g. several days, months or even years, where the person can live a normal life without having to pay more attention to the monitor than necessary with a pair of glasses or a hearing aid. The monitoring may be for purposes of surveillance of a condition of the person and/or for providing some kind of alarm or information in case predetermined conditions are met. The monitor may also be applied for collection of data for further analysis, e.g. for diagnostic purposes or for research use.

An example of monitoring bio-electrical signals is the recording and analysing of an EEG signal for various diagnostic purposes. WO 2006/047874 A1 describes measurement of brain waves particularly for detecting the onset of an epileptic seizure.

EEG monitors may also be applied for surveillance of persons having diabetes, where low blood sugar levels may cause hypoglycaemic attacks. A system for surveillance of the EEG signal where changes may indicate an imminent hypoglycaemic attack is disclosed in WO 2006/066577 A1.

Especially for long term monitoring, it is known to make use of implants to pick up the bio-electrical signals. These implants feature a transmission unit to send the picked up signals to a recording unit that resembles the actual monitoring device. Such a system is known for example from WO 2015/144214 A1.

The implant in such systems is regularly provided with energy by the monitoring device via wireless energy transmission. In order to keep the energy need by the implant low, a transmission distance, that is as short as possible, is the target. Therefore, the monitoring device is usually attached directly over the implant, which in turn may preferably be placed extracranially, having only the skin between the implant and the monitoring device.

Furthermore, the monitoring device should be as small as possible in order to be as discreet as possible when worn. The energy for the own operation as well as or the implant is usually stored in a battery, in most cases a secondary battery cell or "accumulator". In order to charge the battery the monitoring device usually has to be removed from the body, in most cases at a daily interval. In the case where the monitoring device is attached adhesively to the skin, that attachment and detachment causes irritated skin.

Other monitoring devices to be applied to the skin of the patient are known from documents WO 2020/023681 A1, US 2012/022346 A1 and US 2011/319729 A1.

Therefore, there is a need for retaining a monitoring device with as little irritation of the skin as possible.

Such need is met according to the invention by a retaining device according to the features of claim 1. Furthermore, such need is met according to the invention by a wearable monitoring system according to the features of claim 16.

Further expedient and per se inventive embodiments and developments are disclosed in the dependent claims and the following specification.

The retaining device according to the invention is adapted to retain a wearable (especially personal) monitoring device that serves for gathering a bio-electrical signal to a body region of a person. The retaining device comprises a holding frame having a baseplate. The holding frame also comprises retaining means for reversibly retaining the monitoring device onto the baseplate. Hereby, an area covered by the baseplate is reduced with respect to an area covered by the monitoring device. That reduction of that area is intended for ventilation of the skin during wearing the monitoring device.

The term "area covered by..." is understood here and in the following to mean especially the area of the respective part (here especially the baseplate and the monitoring device) projected onto the underlying surface. During intended use that surface would be especially the skin of the user. For the sake of shortness that area is also called "projected area" in the following.

Due to the ventilation of the skin by means of the reduced projected area of the baseplate it is possible to reduce skin irritation even for long term applications. Furthermore, due to the retaining means for reversibly retaining the monitoring device to the body of the user, the monitoring device, that is preferably driven by a secondary battery cell (i. e. a rechargeable battery), may be detached from the body for charging, servicing, readout or else without detaching an adhesive layer from the skin of the user. Such adhesive layer is expediently used for fixation of the holding frame on the skin of the user. For monitoring 24 hours a day, the user may have at least two monitoring devices (or twice the number needed to be worn at the same time), so that at least one monitoring device may be recharged, while the other one monitoring device is in use (i. e. is worn).

In a preferred embodiment a ventilated area which is not covered by (especially massive parts of) the baseplate has at least a portion of 25 percent with respect to the area covered by the monitoring device (i. e. the projected area of the monitoring device). That ventilated area is for example the difference of the projected area of the monitoring device and the projected area of the baseplate. Expediently, the ventilated area is at least 25 percent of the projected area of the monitoring device and, preferably, a maximum of 85 percent. More preferred, the ventilated area is between 30 and 80 percent of the projected area. Expediently, that ratio is between 40 and 75 percent, more expediently between 50 and 70 percent and most preferred at about (i.e. +/- 5 percent) 55 percent.

In an appropriate embodiment the baseplate has a (outer) shape that is at least roughly approximated to the outer contour of the monitoring device. At least roughly approximated is to be understood to mean especially that a part, preferably a major part (i. e. more than 50 percent of the area covered by the baseplate) exhibits an outer shape that corresponds to the monitoring device.

According to the invention, the holding frame has a number of apertures penetrating (or perforating) the baseplate for ventilation of the skin.

Expediently, the number of apertures is more than one.

For example, (described from another perspective) the relation of the projected area of the whole baseplate (i.e. especially without consideration of the apertures) to the ventilated area, in that embodiment preferably the area of the number of apertures, is (during intended use) between 6 and 1,5, in particular between 5,5 and 1,5, expediently between 3 and 1,5, preferably between 2,25 and 1,8, especially between 2,1 and 1,9. For example, the area covered by the baseplate is about 720 mm² and the area of the number apertures is about 130 mm². Thus, the area of the number of (preferably more than one) apertures is especially as large as possible while also maintaining mechanical stability of the baseplate.

In one example the baseplate is perforated by multiple (especially likewise) holes, e.g. circular bores.

According to another embodiment the baseplate has a skeleton-like or meshed structure of interconnected bars (or "webs") outlining the number of apertures. Specifically, the apertures, in that embodiment, are of an oval and/or polygonal shape.

In the sense of an easy and cost effective manufacturing method (especially for a serial production), the holding frame is injection moulded from a thermoplastic material, e. g. a polycarbonate.

For example, the baseplate is just resembled by a relatively narrow ring-like frame structure or ring-like beam having a curvature corresponding to the monitoring device and outlining one aperture. For further stabilization, a further development of that example might have two apertures by means of an additional narrow beam (or web) spanning from side to side of the ring-like beam (i.e. subdividing one bigger aperture into said two apertures).

In an expedient embodiment, the retaining device, furthermore, comprises an adhesive layer (especially the one mentioned above) that is attached at least to a part of the baseplate. Said adhesive layer serves for fixation of the holding frame onto the skin of the person.

According to a preferred embodiment said adhesive layer has a multiple layer structure comprising a carrier layer attached by an adhesive sublayer to the baseplate and a contact layer that serves as a skin adhesive during intended use. Especially, the adhesive layer is resembled by some kind of skin plaster which - by itself - is glued to the baseplate (by way of said adhesive sublayer). In a preferred version, the adhesive layer is of the kind of a usual sensitive skin plaster in order to reduce skin irritation only by wearing the plaster itself.

According to an expedient embodiment also said adhesive layer has a number of through-holes corresponding to the respective aperture of the baseplate. By that, the ventilation of the skin and, thus, the reduction of skin irritation may be enhanced. The through-holes may be (especially round) holes ("bores") that are located within the area of the respective apertures of the baseplate they are aligned with. In that case, the through-holes are smaller than the respective aperture, thus, the area adhering to the skin is enlarged in comparison to the area of the "massive" parts (i.e. without the apertures) of the baseplate. The trough-holes are optionally made by cutting or punching the adhesive layer. Optionally, the through-holes have the same form as the respective aperture.

According to the invention, the baseplate comprises means for ventilation of at least some of the number of apertures especially for the case that the monitoring device is attached to the holding frame. In that case, the monitoring device usually covers the number of apertures at least partially. These means for ventilation are located in a preferred version at an upper side of the baseplate facing the monitoring device during intended use, thus, facing away from the adhesive layer, if applicable.

According to the invention, the means for ventilation described above comprise at least one ventilation channel embossed especially into the upper side of the baseplate. Said at least one channel connects one of the number of apertures with a rim of the baseplate. In other words, said channel enables a sideways ventilation. During intended use of the monitoring device and of the retaining device the channel allows air to flow between the baseplate and the monitoring device to and from the aperture connected by the channel. Thus, the area under the monitoring device may be ventilated since the side of the monitoring device facing the user's skin is in fluid communication with the environment via the at least one ventilation channel. "Embossing", in that case, is considered as locally reducing the wall thickness of the baseplate, especially irrespective of the manufacturing method.

According to a further embodiment the means for ventilation comprise (alternatively or additionally to the above ventilation channel) at least one protrusion serving as a spacer between the surface of the upper side and the surface of the monitoring device being directed to the baseplate during intended use. Preferably, there are more, e. g. two or three such protrusions.

In another expedient embodiment - alternatively or additionally to the aforementioned aperture(s) - the baseplate has a beam-like structure connecting the retaining means and leaving a number of recesses open to a border (or margin) defined by the projected area of the monitoring device. In other words, the beam-like structure has some kind of stem or twig which is relatively narrow and which is running between a number of gaps (the above recesses) that are sideways open. Thus, in a most rudimentary version there is only one beam which is narrow with respect to the width of the monitoring device (e.g. at most about half of the width of the monitoring device). The parts of the projected area of the monitoring device not covered by said beam resemble said gaps - or said aforementioned ventilated area.

According to an expedient embodiment the retaining means for retaining the monitoring device to the baseplate comprise a snap-on hook. Said snap-on hook is preferably located such that a user, i.e. the person wearing the monitoring device, is able to reach it for releasing the monitoring device. Especially in the case of an injection moulded holding frame, a snap-on hook is an easy to design and easy to use retaining mechanism. Additionally, a snap-on hook offers a haptic and/or acoustic feedback of reaching the intended locking state. Preferably, in that case, the monitoring device has an indentation that is designed correspondingly to the snap-on hook such that the latter may snap into that indentation when the monitoring device is positioned in the intended wearing position.

According to another expedient embodiment (especially as an alternative to the snap-on hook) the retaining means comprise a holding magnet. Said holding magnet is adapted to couple with a counter magnet located with the monitoring device.

According to a further expedient embodiment the retaining means comprise (especially additionally to the snap-on hook or the holding magnet) an overhanging structure into the clearing of which the monitoring device is inserted during intended use. Especially, said overhanging structure serves as a counter support or down holder acting together with the snap-on hook or the holding magnet. In that case the snap-on hook or the holding magnet and the overhanging structure are allocated in the regions of opposite ends of the baseplate.

Said overhanging structure resembles in one embodiment a shoe cap like structure. In an alternative embodiment the overhanging structure is made as some kind of rail for guiding the monitoring device over a longer distance than the shoe cap like version.

Preferably, the overhanging structure is positioned in a region of the baseplate facing upwards during intended use. That enables a protection against unintended entangling with a garment, e.g. a pullover during dressing.

According to a further preferred embodiment the snap-on hook is provided at a rim confining a heel part of the baseplate, i. e. especially the part opposite of the part having the overhanging structure. Said heel part is especially tapered with respect to a remaining main part of the base plate. Such tapering enables a higher flexibility of the heel part and, thus, of the snap-on hook.

Especially, the holding frame is roughly designed as the "colour" spades of a card game or as an arrowhead. The "stem" part of the spades being the heel part and the leaves or heart like part being the main part of the baseplate. According to one embodiment the tip part of the spades has the shoe cap like structure of the overhanging structure.

Thus, the outer shape of the baseplate and, due to that, of the holding frame are approximated to a triangle. That is, especially, since the monitoring device is itself also triangular.

According to a further expedient embodiment the retaining device additionally comprises means for preventing an unintended allocation of the monitoring device to the holding frame. In other words, there is a fool proof attaching mechanism with the retaining device.

Said means for preventing an unintended allocation of the monitoring device preferably comprise the baseplate having a curvature. Especially, such curvature is a convex bulge of the baseplate towards the monitoring device. Preferably, such curvature is adapted to a curvature of the monitoring device. In that case, a wrong allocation of the monitoring device would prevent the snap-on hook and/or the overhanging structure from getting a hold on the monitoring device.

According to an alternative (or even additional) embodiment the means for preventing an unintended allocation of the monitoring device comprise the snap-on hook having a length shorter than the thickness of the monitoring device. Additionally, the length of the snap-on hook is different to half of the thickness of the monitoring device. In other words, for preventing the unintended allocation, the snap-on hook is expediently shorter than half of the thickness of the monitoring device or longer than half of the thickness but shorter than the whole thickness. Preferably, the above mentioned indentation of the monitoring device is positioned correspondingly on the outside of the monitoring device. Further preferably, the snap-on hook is expediently shorter than half of the thickness of the monitoring device so that the snap-on hook and the indentation of the monitoring device will not be able to engage with each other if they are not placed correctly. Due to the above described length of the snap-on hook a flipped allocation of the monitoring device will not be possible because the length of the snap-on hook does not match to the indentation of the monitoring device in the latter case.

The wearable monitoring system according to the invention comprises the above described retaining device as well as the above described wearable monitoring device. Therefore, the monitoring system shows the same features and advantages as described above.

Hereinafter, embodiments of the invention are explained on the basis of a drawing. Therein is shown in
- Fig. 1: in a perspective view schematically a retaining device for retaining a wearable monitoring device,
- Fig. 2: in another perspective view schematically the retaining device in an intended use state,
- Fig. 3: in a view upon an upper side an adhesive layer of the retaining device,
- Fig. 4: in a view according to Fig. 3 the retaining device in a state as in Fig. 2,
- Fig. 5: in a view according to Fig. 3 the retaining device and the monitoring device attached to the retaining device,
- Fig. 6: in a sectional view the retaining device and the monitoring device,
- Fig. 7: in another perspective view schematically a further embodiment of the retaining device, and
- Fig. 8-12: in a view according to Fig. 3, respectively, yet further embodiments of the retaining device.

Same features are named by same reference numerals throughout all figures.

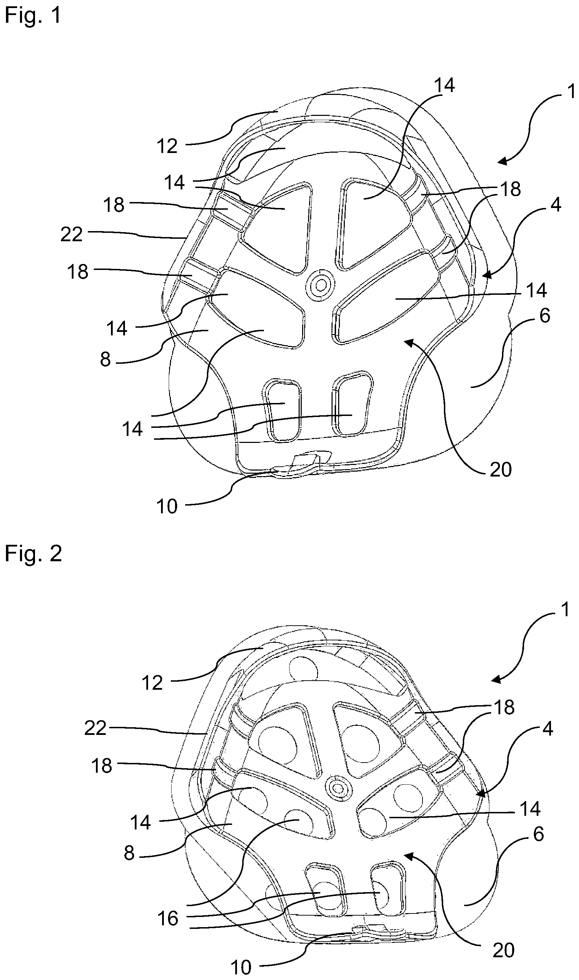

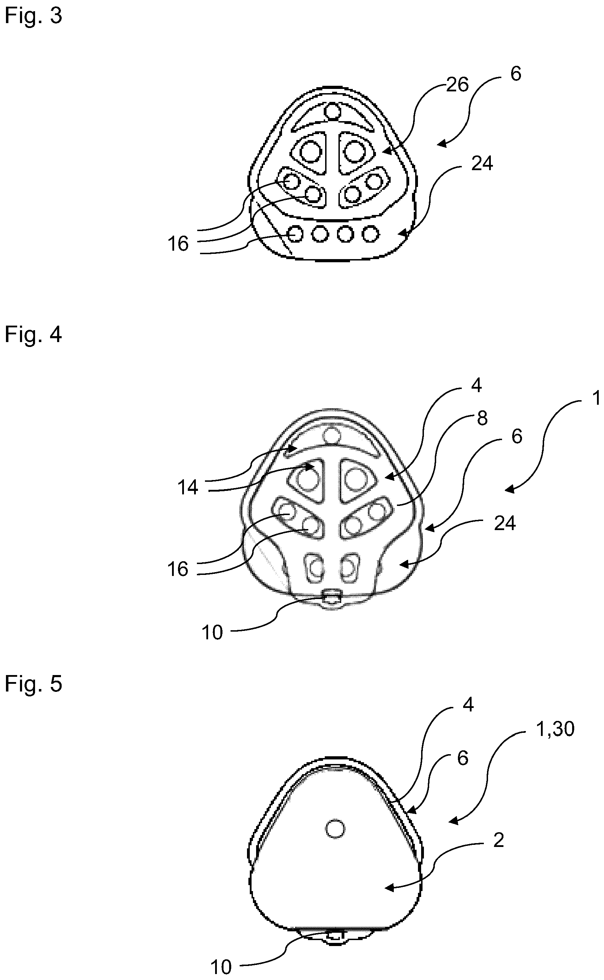

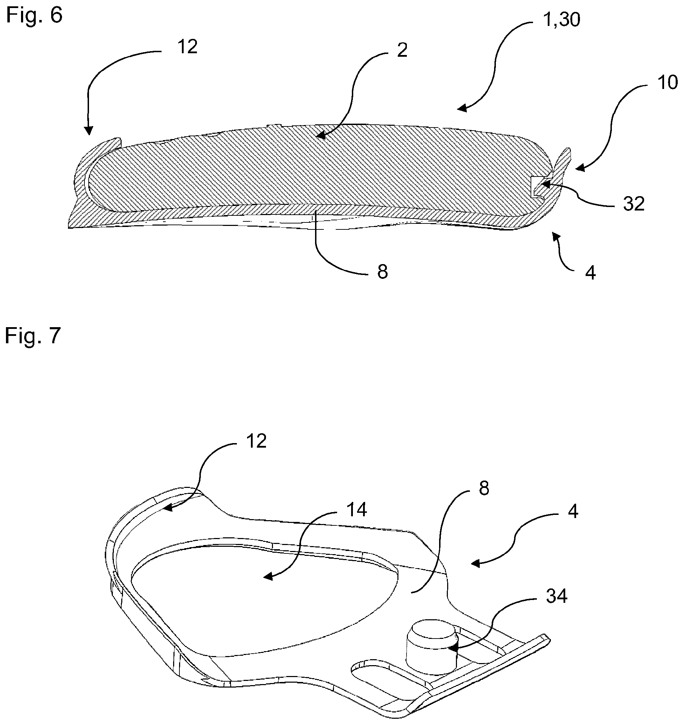

In Fig. 1 a retaining device 1 for a wearable monitoring device 2 (s. Fig. 5 and 6) is shown. The monitoring device 2 is adapted to gather a bio-electrical signal, especially from a subcutaneous implant (not shown). The retaining device 1 is adapted to be worn on the skin over a long term that may be several days.

The retaining device 1 comprises a holding frame 4 for holding (retaining) the monitoring device 2 and an adhesive layer 6 for attachment of the holding frame 4 to the skin of a user. The holding frame 4 comprises a baseplate 8 that has roughly the shape of an arrowhead or spades. In addition to the baseplate 8 the holding frame 4 comprises means for holding the monitoring device 2.

According to the embodiment shown in Fig. 1 the means for holding the monitoring device 2 are resembled by a snap-on hook 10 and an overhanging structure 12. The latter is located at a tip of the arrowhead shape of the baseplate 8 which is worn during intended use facing upwards. The overhanging structure 12 resembles a kind of a shoe cap. The monitoring device 2 which has the shape of a triangle is pushed under the overhanging structure 12 with its tip and is then clipped onto the baseplate 8 by the snap-on hook 10.

The holding frame 4 is manufactured integrally with the baseplate 8, the overhanging structure 12 and the snap-on hook 10 by injection molding, e. g. using a polycarbonate.

In order to further the elastic features of the holding frame 4 in the region of the snap-on hook 10 the baseplate 8 is tapered (just as the foot of the arrowhead) in comparison to the upper (or main) part of the baseplate 8.

The baseplate 8 is perforated by a number of apertures 14. In the embodiment according to Fig. 1 there are seven apertures 14. The apertures 14 serve for ventilation of the underlying skin during usage of the retaining device 1 and of the monitoring device. Therefore, the adhesive layer 6 is also perforated by through-holes 16 that are located in the regions of the apertures 14 (s. Fig. 2). In Fig. 1 the through-holes 16 are not shown because the adhesive layer 6 is not ready for usage and, therefore, covered by a peel-off film (not shown).

Since the monitoring device 2 would cover most parts of the apertures 14 further means for ventilation are formed into the holding frame 4. These means are ventilation channels 18 that lead on an upper side 20 of the baseplate 8 from a rim 22 of the baseplate 8 to a respective aperture 14. The ventilation channels 18 are engraved into the upper side 20 of the baseplate.

A ventilated area, i.e. the difference of the projected area of the monitoring device 2 and the projected area of the "massive parts" of the baseplate 8 (i. e. without the area of the respective apertures 14) is about 472 mm². The projected area of the monitoring device is about 893 mm². Thus, the ventilated area is about 53 percent of the projected area of the monitoring device 2.

In Fig. 3 the adhesive layer 6 is schematically shown. The adhesive layer 6 is made up from a usual plaster 24, having for example a textile layer (woven or nonwoven, not shown in detail) that is carrying a skin adhesive which is covered before intended use by above peel-off film. On the upper side, the adhesive layer 6 comprises an adhesive film 26 (or sublayer) by which it is attached to the baseplate 8, at least to the upper part of the baseplate 8. That is shown in Fig .4. The tapered part (or "heel part") of the baseplate 8 is not bonded to the adhesive layer 6 via the adhesive film 26, thus, enhancing the flexibility of the adhesive layer 6, especially for conforming to the shape of the user's head.

Fig. 5 depicts schematically the monitoring system 30 made up from the retaining device 1 and the monitoring device 2 attached to it.

Fig. 6 depicts in a sectional view schematically the holding frame 4 and the monitoring device 2 in the intended use state. As it can be seen, the tip (left side of Fig. 6) of the monitoring device 2 is held beneath the overhanging structure 12. The "heel" of the monitoring device 2 is held by the snap-on hook 10 engaging in an indentation 32 of the monitoring device 2. The indentation 32 is within a lower half of the thickness of the monitoring device. The snap-on hook 10 is correspondingly short.

Furthermore, the monitoring device 2 as well as the holding frame 4 are curved. Due to that, a foolproof alignment of the monitoring device 2 is made possible. Additionally, the holding frame 4 may fit closely to the skin since the skin especially on the head of a user is not plane by nature.

Fig. 7 schematically shows a further embodiment of the holding frame 4. Here, a holding magnet 34 is used instead of the snap-on hook 10. Furthermore, there is one relatively big aperture 14 and two smaller apertures 14 beside the holding magnet 34. The ventilated area in that embodiment is about 652 mm², and, thus, the ventilated area is 73 percent of the projected area of the monitoring device 2 (the projected area of the monitoring devices 2 of the respective embodiments stays the same).

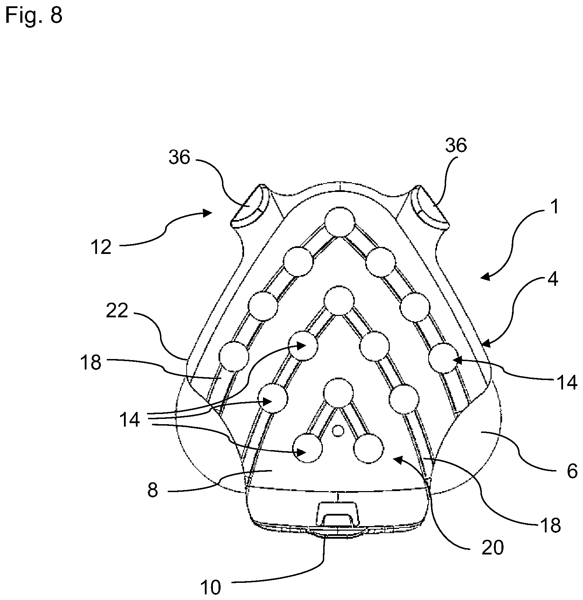

Fig. 8 schematically shows yet a further embodiment of the holding frame 4. In general, this embodiment is similar to the embodiment of Fig. 1 and 2. However, here, the overhanging structure 12 is made of two equal parts on both sides of the tip. Thus, the overhanging structure 12 looks some kind of a pair of floppy ears 36 wherein the monitoring device 30 may be pushed beneath the tips of both floppy ears 36.

Furthermore, the apertures 14 are made circular and are aligned on a row along a respective ventilation channel 18 that connects several apertures 14 with the rim 22 of the holding frame 4.

The ventilated area in that embodiment is about 510 mm², and, thus, the ventilated area is about 57 percent of the projected area of the monitoring device 2.

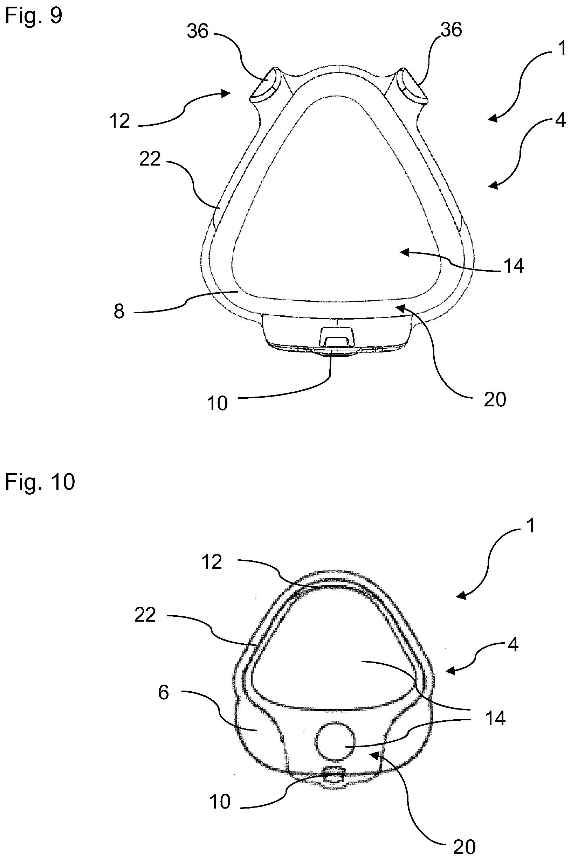

Fig. 9 displays another embodiment of the holding frame 4. The overhanging structure 12 is similar to the embodiment of Fig. 8. The baseplate 8 approximates the shape of the monitoring device 2. However, there is only one (large) aperture 14 that reduces the baseplate 8 to be only a narrow frame beam of a roughly triangular shape that surrounds the aperture 14 ring-like. In that embodiment, the ventilated area is about 72 percent of the projected area of the monitoring device 2.

Fig. 10 shows another embodiment of the holding frame 4. The outer shape of the baseplate 8 corresponds to the one of Fig. 3, but having only two apertures 14, one similar to the one of Fig. 9. The other one being a circular bore in the tapered part (or "heel part") of the baseplate 8. In that embodiment, the ventilated area is also about 72 percent of the projected area of the monitoring device 2.

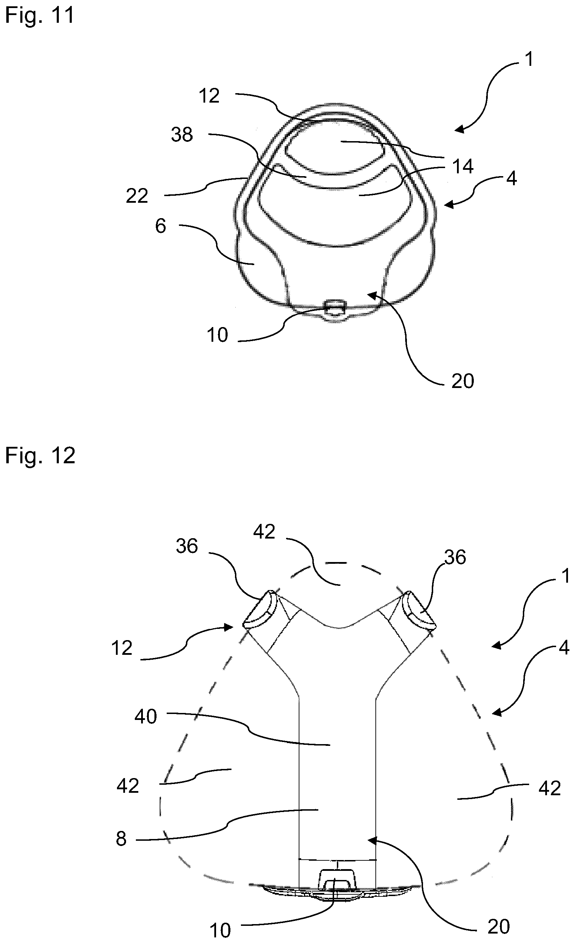

In a yet another embodiment, shown in Fig. 11, a narrow beam or web 38 runs from side to side of the narrow frame beam which is similar to the one of Fig. 9 such that two apertures 14 are shaped. In that embodiment, the ventilated area is about 66 percent of the projected area of the monitoring device 2.

Fig. 12 shows another alternative embodiment of the baseplate 8. The baseplate 8 resembles a stem 40 (or a "central web") connecting the overhanging structure 12 and the snap on hook 10. Spoken figuratively, the baseplate 8 is of a tree-like shape having the stem 40 branching into two "twigs" connection the floppy ears 36. In that case, the ventilation is realized by the reduced area of the baseplate 8 with respect to the area of the monitoring device 2 (indicated by a dashed line). The stem 40 (and its twigs connecting the floppy ears 36) run in other words between gaps 42 (or recesses) that are open to the outside with respect to the outer shape of the monitoring device 2. In that embodiment, the ventilated area is about 73 percent of the projected area of the monitoring device 2.

The above mentioned absolute values of the respective projected areas resemble only exemplary values and are only meant for the better understanding of the relation between projected areas of the apertures 14 and the projected area of the monitoring device.

The subject of the invention is not limited to the embodiments described above, but rather defined by the appended claims.

### List of reference numerals

- 1: retaining device
- 2: monitoring device
- 4: holding frame
- 6: adhesive layer
- 8: baseplate
- 10: snap-on hook
- 12: overhanging structure
- 14: aperture
- 16: through-hole
- 18: ventilation channel
- 20: upper side
- 22: rim
- 24: plaster
- 26: adhesive film
- 30: monitoring system
- 32: indentation
- 34: holding magnet
- 36: floppy ear
- 38: web
- 40: stem
- 42: gap

## Claims

1. Retaining device (1) for retaining a wearable monitoring device (2) for gathering a bio-electrical signal from a body region of a person, comprising a holding frame (4) having a baseplate (8), a number of apertures (14) penetrating the baseplate (8), and retaining means (10,12) for reversibly retaining the monitoring device (2) onto the baseplate (8),
wherein an area covered by the baseplate (8) is reduced with respect to an area covered by the monitoring device (2) for ventilation of the skin during wearing the monitoring device (2),
wherein the baseplate (8) comprises means for ventilation (18) of at least some of the number of apertures (14) that are located at an upper side (20) of the baseplate (8) facing the monitoring device (2) during intended use, **characterised in that**
the means for ventilation comprise at least one ventilation channel (18) embossed into the upper side (20) of the baseplate (8) and connecting one of the number of apertures (14) with a rim (22) of the baseplate (8).

2. Retaining device (1) according to claim 1,
wherein the baseplate (8) has a skeleton-like or meshed structure of interconnected bars outlining the number of apertures (14).

3. Retaining device (1) according to claim 1 or 2,
wherein the means for ventilation comprise at least one protrusion serving as a spacer between the surface of the upper side (20) and the surface of the monitoring device (2) being directed to the baseplate (8) during intended use.

4. Retaining device (1) according to one of claims 1 to 3,
wherein the baseplate (8) has a beam-like structure connecting the retaining means (10,12) leaving a number of recesses open to a border defined by the area covered by the monitoring device (2).

5. Retaining device (1) according to one of claims 1 to 4,
wherein a ventilated area which is not covered by the baseplate (8) has at least a portion of 25 percent with respect to the area covered by the monitoring device (2) and a maximum portion of 85 percent, especially a portion between 40 and 75 percent.

6. Retaining device (1) according to one of claims 1 to 5,
wherein the retaining means comprise a snap-on hook (10).

7. Retaining device (1) according to one of claims 1 to 5,
wherein the retaining means comprise a holding magnet (34).

8. Retaining device (1) according to one of claims 1 to 7,
wherein the retaining means comprise an overhanging structure (12) **into** the clearing of which the monitoring device (2) is inserted during intended use.

9. Retaining device (1) according to claim 8,
wherein the overhanging structure (12) is positioned in a region of the baseplate (8) facing upwards during intended use.

10. Retaining device (1) according to one of claims 6to 9,
wherein the snap-on hook (10) is provided at a rim confining a heel part of the baseplate (8) being tapered with respect to a remaining main part of the baseplate (8).

11. Retaining device (1) according to one of claims 1 to 10,
comprising means for preventing an unintended allocation of the monitoring device (2) to the holding frame (4).

12. Retaining device (1) according to claim 11,
wherein the means for preventing an unintended allocation of the monitoring device (2) comprise the baseplate (8) having a curvature, especially the curvature being adapted to a curvature of the monitoring device (2).

13. Retaining device (1) according to claim 11 or 12,
wherein the means for preventing an unintended allocation of the monitoring device (2) comprise the snap-on hook (10) having a length shorter than the thickness of the monitoring device (2) and different to half of the thickness of the monitoring device (2).

14. Retaining device (1) according to one of claims 1 to 13,
an adhesive layer (6) attached at least to a part of the baseplate (8) for fixation of the holding frame (4) onto the skin of the person, especially wherein the adhesive layer (6) has a multiple layer structure comprising a carrier layer attached by an adhesive sublayer to the baseplate (8) and a contact layer that serves as a skin adhesive during intended use.

15. Retaining device (1) according to claim 14,
wherein the adhesive layer (6) has a number of through-holes (16) corresponding to the respective aperture (14) of the baseplate (8).

16. Wearable monitoring system (30) comprising a wearable monitoring device (2) for gathering a bio-electrical signal and a retaining device (1) according to one of claims 1 to 15.

## Patentansprüche

1. Haltevorrichtung (1) zum Halten einer tragbaren Überwachungsvorrichtung (2) zum Erfassen eines bioelektrischen Signals aus einer Körperregion einer Person, umfassend einen Halterahmen (4) mit einer Grundplatte (8), eine Anzahl von Öffnungen (14), die die Grundplatte (8) durchdringen und Haltemittel (10, 12) zum reversiblen Halten der Überwachungsvorrichtung (2) auf der Grundplatte (8),
wobei eine von der Grundplatte (8) bedeckte Fläche im Vergleich zu einer von der Überwachungsvorrichtung (2) bedeckten Fläche zur Belüftung der Haut während des Tragens der Überwachungsvorrichtung (2) verkleinert ist,
wobei die Grundplatte (8) Mittel (18) zur Belüftung mindestens einiger der Anzahl von Öffnungen (14) umfasst, die sich an einer Oberseite (20) der Grundplatte (8) befinden, die der Überwachungsvorrichtung (2) während des bestimmungsgemäßen Gebrauchs zugewandt ist,
**dadurch gekennzeichnet, dass**
die Belüftungsmittel mindestens einen Belüftungskanal (18) umfassen, der in die Oberseite (20) der Grundplatte (8) eingeprägt ist und eine der Anzahl von Öffnungen (14) mit einem Rand (22) der Grundplatte (8) verbindet.

2. Haltevorrichtung (1) nach Anspruch 1,
wobei die Grundplatte (8) eine skelettartige oder netzartige Struktur aus miteinander verbundenen Stäben aufweist, die die Anzahl von Öffnungen (14) umranden.

3. Haltevorrichtung (1) nach Anspruch 1 oder 2,
wobei die Belüftungsmittel mindestens einen Vorsprung umfassen, der als Abstandshalter zwischen der Oberfläche der Oberseite (20) und der Oberfläche der Überwachungsvorrichtung (2) dient und bei bestimmungsgemäßem Gebrauch auf die Grundplatte (8) gerichtet ist.

4. Haltevorrichtung (1) nach einem der Ansprüche 1 bis 3,
wobei die Grundplatte (8) eine balkenartige Struktur aufweist, die die Haltemittel (10, 12) verbindet, wobei eine Anzahl von Aussparungen zu einem Rand offen bleibt, der durch die vor Überwachungsvorrichtung (2) abgedeckte Fläche definiert ist.

5. Haltevorrichtung (1) nach einem der Ansprüche 1 bis 4,
wobei eine belüftete Fläche, die nicht von der Grundplatte (8) bedeckt ist, mindestens einen Anteil von 25 Prozent der von der Überwachungsvorrichtung (2) bedeckten Fläche und einen maximalen Anteil von 85 Prozent, insbesondere einen Anteil zwischen 40 und 75 Prozent, aufweist.

6. Haltevorrichtung (1) nach einem der Ansprüche 1 bis 5,
wobei die Haltemittel einen Schnapphaken (10) umfassen.

7. Haltevorrichtung (1) nach einem der Ansprüche 1 bis 5,
wobei die Haltemittel einen Haltemagneten (34) umfassen.

8. Haltevorrichtung (1) nach einem der Ansprüche 1 bis 7,
wobei die Haltemittel eine überhängende Struktur (12) umfassen, in deren Freiraum die Überwachungsvorrichtung (2) bei bestimmungsgemäßem Gebrauch eingesetzt wird.

9. Haltevorrichtung (1) nach Anspruch 8,
wobei die überhängende Struktur (12) in einem Bereich der Grundplatte (8) positioniert ist, der bei bestimmungsgemäßem Gebrauch nach oben zeigt.

10. Haltevorrichtung (1) nach einem der Ansprüche 6 bis 9,
wobei der Schnapphaken (10) an einem Rand bereitgestellt ist, der einen Fersenbereich der Grundplatte (8) umschließt, der sich gegenüber einem übrigen Hauptteil der Grundplatte (8) verjüngt.

11. Haltevorrichtung (1) nach einem der Ansprüche 1 bis 10,
umfassend Mittel zum Verhindern einer unbeabsichtigten Zuordnung der Überwachungsvorrichtung (2) zum Halterahmen (4).

12. Haltevorrichtung (1) nach Anspruch 11,
wobei die Mittel zum Verhindern einer unbeabsichtigten Zuordnung der Überwachungsvorrichtung (2) die Grundplatte (8) mit einer Krümmung umfassen, wobei die Krümmung insbesondere an eine Krümmung der Überwachungsvorrichtung (2) angepasst ist.

13. Haltevorrichtung (1) nach Anspruch 11 oder 12,
wobei die Mittel zum Verhindern einer unbeabsichtigten Zuordnung des Überwachungsvorrichtung (2) den Schnapphaken (10) umfassen, der eine Länge kürzer als die Dicke der Überwachungsvorrichtung (2) und ungleich der Hälfte der Dicke der Überwachungsvorrichtung (2) aufweist.

14. Haltevorrichtung (1) nach einem der Ansprüche 1 bis 13,
eine Klebeschicht (6), die zumindest an einem Teil der Grundplatte (8) zur Fixierung des Halterahmens (4) auf der Haut der Person angebracht ist, insbesondere wobei die Klebeschicht (6) einen mehrschichtigen Aufbau aufweist, umfassend eine Trägerschicht, die mittels einer Klebeteilschicht an der Grundplatte (8) befestigt ist, und eine Kontaktschicht, die während des bestimmungsgemäßen Gebrauchs als Hautkleber dient.

15. Haltevorrichtung (1) nach Anspruch 14,
wobei die Klebeschicht (6) eine Anzahl von Durchgangslöchern (16) aufweist, die der jeweiligen Öffnung (14) der Grundplatte (8) entsprechen.

16. Tragbares Überwachungssystem (30), umfassend eine tragbare Überwachungsvorrichtung (2) zum Erfassen eines bioelektrischen Signals und eine Haltevorrichtung (1) nach einem der Ansprüche 1 bis 15.

## Revendications

1. Dispositif de retenue (1) destiné à retenir un dispositif de surveillance portable (2) pour recueillir un signal bioélectrique provenant d'une région du corps d'une personne, comprenant un cadre de maintien (4) doté d'une plaque de base (8), d'un certain nombre d'ouvertures (14) traversant la plaque de base (8) et de moyens de retenue (10, 12) permettant de retenir de manière réversible le dispositif de surveillance (2) sur la plaque de base (8),
dans lequel une surface couverte par la plaque de base (8) est réduite par rapport à une surface couverte par le dispositif de surveillance (2) pour la ventilation de la peau pendant le port du dispositif de surveillance (2),
dans lequel la plaque de base (8) comprend des moyens de ventilation (18) d'au moins une partie des ouvertures (14) situées sur une face supérieure (20) de la plaque de base (8) faisant face au dispositif de surveillance (2) pendant l'utilisation prévue,
**caractérisé en ce que**
les moyens de ventilation comprennent au moins un canal de ventilation (18) gaufré dans la face supérieure (20) de la plaque de base (8) et reliant l'une des ouvertures (14) à un rebord (22) de la plaque de base (8).

2. Dispositif de retenue (1) selon la revendication 1,
dans lequel la plaque de base (8) présente une structure squelettique ou maillée de barres interconnectées délimitant le nombre d'ouvertures (14).

3. Dispositif de retenue (1) selon la revendication 1 ou 2,
dans lequel les moyens de ventilation comprennent au moins une protubérance servant d'entretoise entre la surface du côté supérieur (20) et la surface du dispositif de surveillance (2) étant dirigée vers la plaque de base (8) lors de l'utilisation prévue.

4. Dispositif de retenue (1) selon l'une des revendications 1 à 3,
dans lequel la plaque de base (8) présente une structure en forme de poutre reliant les moyens de retenue (10, 12) laissant un certain nombre de cavités ouvertes sur une bordure définie par la zone couverte par le dispositif de surveillance (2).

5. Dispositif de retenue (1) selon l'une des revendications 1 à 4,
dans lequel une zone ventilée qui n'est pas couverte par la plaque de base (8) présente au moins une portion de 25 % par rapport à la surface couverte par le dispositif de surveillance (2) et une portion maximale de 85 %, notamment une portion comprise entre 40 et 75 %.

6. Dispositif de retenue (1) selon l'une des revendications 1 à 5,
dans lequel les moyens de retenue comprennent un crochet à enclenchement (10).

7. Dispositif de retenue (1) selon l'une des revendications 1 à 5,
dans lequel les moyens de retenue comprennent un aimant de maintien (34).

8. Dispositif de retenue (1) selon l'une des revendications 1 à 7,
dans lequel les moyens de retenue comprennent une structure en surplomb (12) dans laquelle le dispositif de surveillance (2) est inséré lors de l'utilisation prévue.

9. Dispositif de retenue (1) selon la revendication 8,
dans lequel la structure en surplomb (12) est positionnée dans une région de la plaque de base (8) orientée vers le haut lors de l'utilisation prévue.

10. Dispositif de retenue (1) selon l'une des revendications 6 à 9,
dans lequel le crochet à enclenchement (10) est prévu sur un rebord confinant une partie talon de la plaque de base (8) qui est conique par rapport à une partie principale restante de la plaque de base (8).

11. Dispositif de retenue (1) selon l'une des revendications 1 à 10,
comprenant des moyens pour empêcher une attribution non intentionnelle du dispositif de surveillance (2) au cadre de maintien (4).

12. Dispositif de retenue (1) selon la revendication 11,
dans lequel les moyens d'empêcher une attribution non intentionnelle du dispositif de surveillance (2) comprennent la plaque de base (8) présentant une courbure, en particulier la courbure étant adaptée à une courbure du dispositif de surveillance (2).

13. Dispositif de retenue (1) selon la revendication 11 ou 12,
dans lequel les moyens d'empêcher une attribution non intentionnelle du dispositif de surveillance (2) comprennent le crochet à enclenchement (10) présentant une longueur plus courte que l'épaisseur du dispositif de surveillance (2) et différente de la moitié de l'épaisseur du dispositif de surveillance (2).

14. Dispositif de retenue (1) selon l'une quelconque des revendications 1 à 13,
une couche adhésive (6) fixée au moins à une partie de la plaque de base (8) pour la fixation du cadre de maintien (4) sur la peau de la personne, en particulier dans lequel la couche adhésive (6) présente une structure multicouche comprenant une couche porteuse fixée par une sous-couche adhésive à la plaque de base (8) et une couche de contact qui sert d'adhésif cutané lors de l'utilisation prévue.

15. Dispositif de retenue (1) selon la revendication 14,
dans lequel la couche adhésive (6) comporte un certain nombre de trous traversants (16) correspondant à l'ouverture respective (14) de la plaque de base (8).

16. Système de surveillance portable (30) comprenant un dispositif de surveillance portable (2) pour recueillir un signal bioélectrique et un dispositif de retenue (1) selon l'une des revendications 1 à 15.
